# EUROPÄISCHE PATENTSCHRIFT

(11) **EP 2 856 447 B1**
(45) Veröffentlichungstag und Bekanntmachung des Hinweises auf die Patenterteilung: **16.01.2019**
(21) Anmeldenummer: 13723811.9
(22) Anmeldetag: 22.05.2013
(51) Int. Cl.: G08B 21/06

(54) **VERFAHREN UND VORRICHTUNG ZUR FAHRERZUSTANDSERKENNUNG**
METHOD AND DEVICE FOR RECOGNISING THE CONDITION OF A DRIVER
PROCÉDÉ ET DISPOSITIF POUR DÉTERMINER L'ETAT D'UN CONDUCTEUR

(30) Priorität: 25.05.2012 DE 102012208822
(43) Veröffentlichungstag der Anmeldung: 08.04.2015
(73) Patentinhaber: Robert Bosch GmbH, 70442 Stuttgart (DE)
(72) Erfinder: SCHERMANN, Ernst, 70771 Leinfelden-Echerdingen (DE); OFFENHAEUSER, Andreas, 71672 Marbach Am Neckar (DE)
(86) Internationale Anmeldenummer: PCT/EP2013/060458
(87) Internationale Veröffentlichungsnummer: WO 2013/174838

(56) Entgegenhaltungen:
- DE-A1- 4 338 244
- DE-A1- 10 359 125
- DE-A1-102005 055 971
- DE-A1-102009 009 975

## Beschreibung

### Stand der Technik

Aus der US6313749B1 sind Verfahren zur Bestimmung der Müdigkeit eines Maschinenbedieners bekannt, die auf einer Auswertung des zirkadianen Rhythmus beruhen. Zur Bestimmung des zirkadianen Rhythmus werden Uhrzeitwerte als Eingangsgrößen verwendet.

Die DE 10 2005 055 971 A1 offenbart eine Bestimmung der Belastung eines Fahrzeugführers auf Grundlage vereinheitlichter Belastungsfaktoren. Ein Belastungsfaktor ist die Witterung, die unter Verwendung der Helligkeit bestimmt wird. Die DE 10 2009 009 975 A1 offenbart ein Verfahren zur Ermittlung eines die Aufmerksamkeit eines Fahrers während einer Fahrt beschreibenden Aufmerksamkeitswertes. Dazu kann die Helligkeit berücksichtigt werden. Die DE 103 59 125 A1 offenbart ein Verfahren zur Erkennung der Reaktionsbereitschaft eines Fahrers eines Kraftfahrzeugs unter Verwendung einer Umgebungshelligkeit. Die DE 43 38 244 A1 offenbart ein Gefahrenvermeidungssystem für ein Fahrzeug. Dabei entscheidet eine Gefahrenerkennungs-Bestätigungseinheit, ob der Fahrer sich eines gefährlichen Fahrmodus bewusst ist.

### Offenbarung der Erfindung

Die Erfindung entspricht einem Verfahren nach Anspruch 1 sowie einer Vorrichtung nach Anspruch 14. Weitere Ausgestaltungen der Erfindung sind in den Unteransprüchen wiedergegeben.

Die Erfindung geht aus von einem Verfahren zur Bestimmung einer den Zustand des Fahrers eines Fahrzeugs repräsentierenden Größe, die aus einer anderen Größe bestimmt wird, die die Helligkeit repräsentiert, der der Fahrer ausgesetzt ist.

Der Kern der Erfindung besteht darin, dass dieses Verfahren uhrzeitunabhängig durchgeführt wird. Zu diesem Zweck wird die Helligkeit mittels eines Sensors bestimmt und aus der so gemessenen Helligkeit wird der Fahrerzustand bestimmt.

Aus medizinischen Untersuchungen ist es bekannt, dass die auf einen Menschen einwirkende Helligkeit einen nennenswerten Einfluss auf seinen Zustand wie beispielsweise die Müdigkeit und/oder die Reaktionsfähigkeit hat.

Die erfindungsgemäß uhrzeitunabhängige Bestimmung der den Fahrerzustand repräsentierenden Größe aus der Helligkeit führt zu dem Vorteil, dass Fehler vermieden werden, die bei von Uhrzeitwerten abhängigen Auswertungen durch falsche Uhrzeitwerte verursacht werden können.
Ein weiterer Vorteil entsteht durch die Vermeidung von Effekten, die in Zusammenhang mit der geographischen Position des Fahrzeugs stehen und die auftreten, wenn beispielsweise mitteleuropäische Korrelationen zwischen Helligkeitswerten und Uhrzeitwerten zur Bestimmung des Fahrerzustands zu Grunde gelegt werden.
Beispielsweise bestehen in Abhängigkeit der geographischen Breite jahreszeitabhängige, unterschiedlich lange Zeitdauern zwischen Sonnenauf- und Sonnenuntergang. Dieser Umstand führt beispielsweise in Teilen Skandinaviens dazu, dass zum einen im Dezember bereits am frühen Nachmittag (gegen 16 Uhr) völlige Dunkelheit herrscht, zum anderen ist es zur Sommersonnenwende auch nachts (gegen 24 Uhr) hell.
Ferner kann es bei Fahrten durch Gebiete, für die über mehrere Längengrade hinweg die gleiche Zeitzone festgelegt ist (beispielsweise Volksrepublik China), ebenfalls zu Unstimmigkeiten zwischen dem tatsächlich durch die Helligkeit bedingten Fahrerzustand und dem auf Grundlage eines Uhrzeitwertes ermittelten Fahrerzustands kommen.
In beiden Fällen könnte eine lediglich auf Uhrzeitwerten beruhende und mitteleuropäische Uhrzeitwert-Helligkeitswert-Korrelationen berücksichtigende Bestimmung des Fahrerzustands nicht optimal sein, da sie dem Umstand nicht Rechnung trägt, dass sich der Fahrer einer für den Uhrzeitwert untypischen oder ungewöhnlichen Helligkeit oder Dunkelheit ausgesetzt sieht.
Gemäß einer vorteilhaften Ausgestaltung der Erfindung kann die den Fahrerzustand repräsentierende Größe direkt aus einer anderen Größe bestimmt werden, die die Helligkeit repräsentiert, der der Fahrer ausgesetzt ist. Gemäß dieser Ausgestaltung können zusätzlich weitere Größen bei der Bestimmung des Fahrerzustands berücksichtigt werden. Der Fahrerzustand wird gemäß dieser Ausgestaltung unter Berücksichtigung mehrerer unabhängiger Komponenten bestimmt, wobei die die Helligkeit repräsentierende Größe direkt zur Bestimmung einer unabhängigen Komponente verwendet werden kann.

In einer weiteren vorteilhaften Ausgestaltung des Verfahrens wird bei der Bestimmung der Helligkeit ein zeitlicher Filter (beispielsweise ein Totzeitglied) verwendet. Die Verwendung eines zeitlichen Filters führt erfindungsgemäß zu dem Vorteil, dass Umstände streckenbaulicher (zum Beispiel Tunnel oder Alleen) oder meteorologischer (zum Beispiel kurze Unwetter) Natur, die zu kurzen und schnell vorübergehenden Zeitspannen mit verminderter Helligkeit führen, berücksichtigt werden. Durch die Verwendung eines zeitlichen Filters ist sichergestellt, dass kurzfristige Änderungen der Helligkeit weniger stark gewichtet werden können als längerfristige Änderungen. Denkbar ist zu diesem Zweck, dass ein zeitlicher Schwellenwert Verwendung findet und erst nach einer längerfristig andauernden Veränderung der Helligkeit der Fahrerzustand als ebenfalls verändert betrachtet wird.

Erfindungsgemäß handelt es sich bei der den Zustand des Fahrers repräsentierenden Größe um die Müdigkeit.
Grund für die Bestimmung der Müdigkeit ist der Umstand, dass insbesondere der Müdigkeitsverlauf des Menschen wesentlich durch die durch ihn wahrnehmbare Helligkeit beeinflusst wird.
Vorzugsweise wird die Helligkeit in Form der Beleuchtungsstärke in Lux gemessen. Durch Messung der Beleuchtungsstärke in Lux ist sichergestellt, dass vergleichbare und physikalisch genau definierte Messwerte als Grundlage zur Berechnung des Fahrerzustands, insbesondere der Müdigkeit verwendet werden können.
Zur Bestimmung der Helligkeit, insbesondere der Beleuchtungsstärke, werden vorzugsweise Lichtsensoren verwendet.

Neben der den Fahrerzustand repräsentierenden Größe können vorteilhafterweise weitere, ebenfalls den Fahrerzustand repräsentierende Größen berücksichtigt werden. Durch die Berücksichtigung dieser weiteren Größen kann die erfindungsgemäße, uhrzeitunabhängige und auf Auswertung der Helligkeit beruhende Größe gewichtet und plausibilisiert werden.

Die vorzugsweisen Ausführungsformen zur Bestimmung weiterer den Fahrerzustand repräsentierenden Größen sind im Folgenden dargestellt.

Vorteilhafterweise geht in die Bestimmung der den Fahrerzustand repräsentierenden Größe das Querführungsverhalten, insbesondere das Lenkverhalten des Fahrers ein. Ferner geht in die Bestimmung der den Fahrerzustand repräsentierenden Größe vorzugsweise das Längsführungsverhalten, insbesondere die Pedalbetätigung des Fahrers ein.

In einer weiteren Ausgestaltung wird vorzugsweise bei der Bestimmung der den Fahrerzustand repräsentierenden Größe das Vermögen des Fahrers berücksichtigt, das Fahrzeug möglichst innerhalb einer Fahrspur zu halten.

Die Auswertung des Fahrverhaltens gehört zum Stand der Technik und stellt eine sehr gute Möglichkeit dar, den Fahrerzustand abzuschätzen.

Vorteilhaft geht in die Bestimmung der den Fahrerzustand repräsentierenden Größe die Betätigung von Bedienelementen, beispielsweise des Blinkers und/oder des Fensterhebers, durch den Fahrer ein.

Vorzugsweise geht in die Bestimmung der den Fahrerzustand repräsentierenden Größe die Interaktion des Fahrers mit dem Fahrzeug über HMI- und Infotainmentsysteme, wie ein integriertes Navigationssystem und/oder eine Sprachsteuerung und/oder eine Sprachausgabe, ein.

Die Bestimmung des Fahrerzustands unter Berücksichtigung der Analyse der Interaktion des Fahrers mit HMI- (Human-Machine Interface, Mensch-Maschine-Schnittstelle) und Infotainmentsystemen bringt zudem den Vorteil mit sich, den Fahrerzustand aktiv, beispielsweise über Reaktionsdauern, zu bestimmen.
In einer weiteren vorteilhaften Ausführung der Erfindung wird bei der Bestimmung der den Fahrerzustand repräsentierenden Größe eine weitere Größe berücksichtigt, die über eine Fahrzeug-Innenraumkamera zur Fahrerbeobachtung ermittelt wird.
Vorzugsweise wird schließlich bei der Bestimmung der den Fahrerzustand repräsentierenden Größe eine andere Größe berücksichtigt, die mindestens einen physiologischen Zustand des Fahrers repräsentiert.
Bei der einen physiologischen Zustand eines Fahrers repräsentierenden Größe die zur Bestimmung der den Fahrerzustand repräsentierenden Größe berücksichtigt wird, handelt es sich vorzugsweise um die Körpertemperatur und/oder um die Herzschlagfrequenz des Fahrers.
Vorteilhaft werden zur Bestimmung der physiologischen Größen Sensoren verwendet, die im Rückspiegel und/oder im Fahrersitz und/oder im Fahrersicherheitsgurt und/oder im Lenkrad angebracht sind.
Insbesondere die mittels der genannten Sensoren ermittelten Daten hinsichtlich des physiologischen Zustands des Fahrers (Krankheit, Stress) sind geeignet, den Fahrerzustand genauer zu bestimmen und den erfindungsgemäß bestimmten Fahrerzustand zu plausibilisieren.
Vorzugsweise wird zur Bestimmung einer den Zustand des Fahrers eines Fahrzeugs repräsentierenden Größe eine Vorrichtung verwendet, mit der die Größe uhrzeitunabhängig wenigstens anhand einer anderen Größe bestimmt wird, die die Helligkeit repräsentiert, der der Fahrer ausgesetzt ist.

### Kurze Beschreibung der Zeichnungen

Im folgenden Abschnitt wird die Erfindung anhand von Ausführungsbeispielen, aus denen sich weitere erfinderische Merkmale ergeben können, auf die die Erfindung aber in ihrem Umfang nicht beschränkt ist, erläutert. Die Ausführungen sind in den Zeichnungen dargestellt.

Es zeigen:
- Fig. 1: eine schematische Darstellung des Zusammenhangs zwischen Müdigkeit und Helligkeit;
- Fig. 2: eine schematische Darstellung der Bestimmung des Fahrerzustands anhand mehrerer unabhängiger Komponenten.

### Ausführungsform der Erfindung

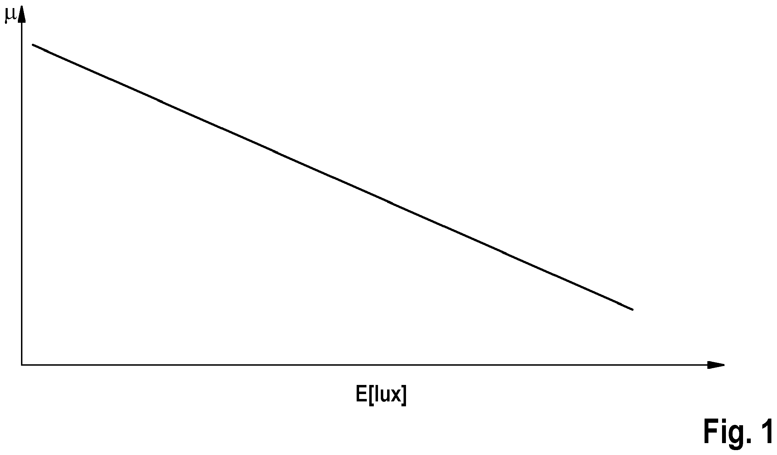

In Figur 1 ist der Zusammenhang zwischen Müdigkeit (µ) und Helligkeit (E) schematisch dargestellt. Aufgetragen ist die Müdigkeit (µ) auf der Ordinate gegenüber der Helligkeit (E) in Lux auf der Abszisse. Dargestellt ist, dass bei zunehmender Helligkeit (E) die Müdigkeit (µ) sinkt. Der Zusammenhang ist linear und wird zu einer direkten Bestimmung der Müdigkeit (µ) aus der Helligkeit (E) heraus verwendet.

Über die Müdigkeit (µ) hinaus, können andere, den Fahrerzustand (FZ) repräsentierende Größen, auf der Ordinate gegenüber der Helligkeit (E) auf der Abszisse aufgetragen werden. Die den Fahrerzustand repräsentierenden Größen würden sich in der Auftragung als von der Helligkeit (E) abhängige, veränderliche Größen darstellen.

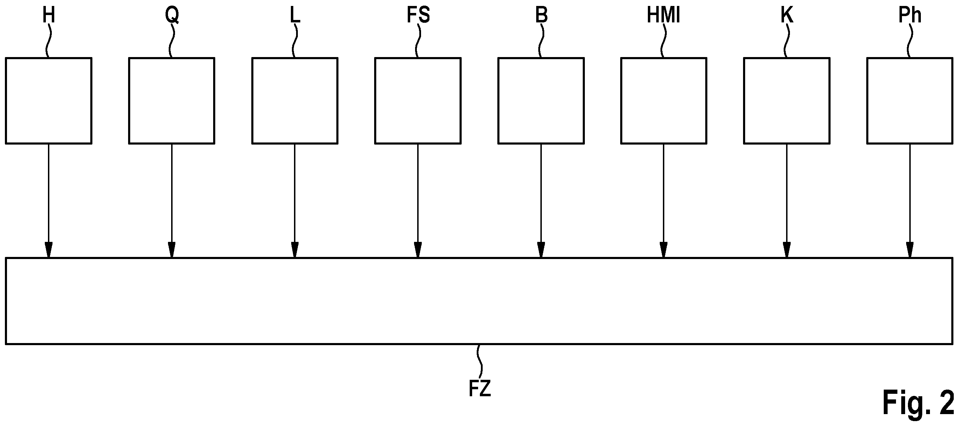

In Figur 2 ist die Bestimmung des Fahrerzustands (FZ) anhand mehrerer unabhängiger Komponenten dargestellt. Vornehmlich wird der Fahrerzustand unter Berücksichtigung einer die Helligkeit repräsentierenden Größe (H) bestimmt. Unabhängig von der Helligkeit können zusätzlich weitere Größen berücksichtigt und zur Bestimmung des Fahrerzustands verwendet werden.
Die weiteren Größen, die bei der Bestimmung des Fahrerzustands (FZ) berücksichtigt werden können, können ermittelt werden aus: dem Querführungsverhalten (Q), insbesondere dem Lenkwinkelverhalten, dem Längsführungsverhalten (L), insbesondere der Betätigung der Pedale, dem Fahrspurverhalten (FS), der Betätigung der Bedienelemente (B), zum Beispiel Blinker und/oder Fensterheber, der Interaktion mit HMI-Systemen (HMI), Daten, die mit der Fahrzeug-Innenraumkamera ermittelt werden, (K) und dem physiologischen Zustand des Fahrers (Ph).

### Bezugszeichenliste:

- µ: Müdigkeit
- E: Helligkeit
- H: Eine die Helligkeit (E) repräsentierende Größe
- Q: Querführungsverhalten
- L: Längsführungsverhalten
- FS: Fahrspurverhalten
- B: Betätigung der Bedienelemente
- HMI: Human-Machine Interface, Mensch-Maschine-Schnittstelle
- K: Daten die mit der Fahrzeug-Innenraumkamera ermittelt werden
- Ph: Physiologischer Zustand
- FZ: Fahrerzustand

## Patentansprüche

1. Verfahren zur Bestimmung einer die Müdigkeit des Fahrers eines Fahrzeugs repräsentierenden Größe, wobei die Größe uhrzeitunabhängig wenigstens anhand einer anderen Größe bestimmt wird, die die Helligkeit repräsentiert, der der Fahrer ausgesetzt ist,
**dadurch gekennzeichnet, dass** die Größe, die die Helligkeit repräsentiert, der der Fahrer ausgesetzt ist, direkt unter Verwendung eines linearen Zusammenhangs zwischen Müdigkeit und Helligkeit zur Bestimmung der den Fahrerzustand repräsentierenden Größe verwendet wird.

2. Verfahren nach Anspruch 1,
**dadurch gekennzeichnet, dass**
die Größe, die die Helligkeit repräsentiert, der der Fahrer ausgesetzt ist, unter Verwendung eines zeitlichen Filters, insbesondere eines Totzeitglieds, bestimmt wird.

3. Verfahren nach Anspruch 1,
**dadurch gekennzeichnet, dass**
die Helligkeit in Form der Beleuchtungsstärke gemessen wird.

4. Verfahren nach Anspruch 1,
**dadurch gekennzeichnet, dass**
zur Bestimmung der Helligkeit Lichtsensoren verwendet werden.

5. Verfahren nach Anspruch 1,
**dadurch gekennzeichnet, dass**
in die Bestimmung der den Fahrerzustand repräsentierenden Größe das Querführungsverhalten, insbesondere das Lenkverhalten des Fahrers eingeht.

6. Verfahren nach Anspruch 1,
**dadurch gekennzeichnet, dass**
in die Bestimmung der den Fahrerzustand repräsentierenden Größe das Längsführungsverhalten, insbesondere die Pedalbetätigung des Fahrers eingeht.

7. Verfahren nach Anspruch 1,
**dadurch gekennzeichnet, dass**
in die Bestimmung der den Fahrerzustand repräsentierenden Größe das Vermögen des Fahrers, das Fahrzeug möglichst innerhalb einer Fahrspur zu halten, eingeht.

8. Verfahren nach Anspruch 1,
**dadurch gekennzeichnet, dass**
in die Bestimmung der den Fahrerzustand repräsentierenden Größe die Betätigung von Bedienelementen, beispielsweise des Blinkers und/oder des Fensterhebers, durch den Fahrer eingeht.

9. Verfahren nach Anspruch 1,
**dadurch gekennzeichnet, dass**
in die Bestimmung der den Fahrerzustand repräsentierenden Größe die Interaktion des Fahrers mit dem Fahrzeug über HMI- und Infotainmentsysteme, wie ein integriertes Navigationssystem und/oder eine Sprachsteuerung und/oder eine Sprachausgabe, eingeht.

10. Verfahren nach Anspruch 1,
**dadurch gekennzeichnet, dass**
in die Bestimmung der den Fahrerzustand repräsentierenden Größe eine weitere Größe eingeht, die über eine Fahrzeug-Innenraumkamera zur Fahrerbeobachtung ermittelt wird.

11. Verfahren nach Anspruch 1,
**dadurch gekennzeichnet, dass**
in die Bestimmung der den Fahrerzustand repräsentierenden Größe eine andere Größe, die mindestens einen physiologischen Zustand des Fahrers repräsentiert, eingeht.

12. Verfahren nach Anspruch 11,
**dadurch gekennzeichnet, dass**
es sich bei den physiologischen Größen des Fahrers um die Körpertemperatur und/oder um die Herzschlagfrequenz handelt.

13. Verfahren nach Anspruch 11,
**dadurch gekennzeichnet, dass**
zur Bestimmung der physiologischen Größen Sensoren verwendet werden, wobei diese Sensoren im Rückspiegel und/oder im Fahrersitz und/oder im Fahrersicherheitsgurt und/oder im Lenkrad angebracht sind.

14. Vorrichtung zur Bestimmung einer die Müdigkeit des Fahrers eines Fahrzeugs repräsentierenden Größe, wobei die Vorrichtung derart ausgestaltet ist, dass die Größe uhrzeitunabhängig wenigstens anhand einer anderen Größe bestimmt wird, die die Helligkeit repräsentiert, der der Fahrer ausgesetzt ist
**dadurch gekennzeichnet, dass** die Vorrichtung derart ausgestaltet ist, dass die Größe, die die Helligkeit repräsentiert, der der Fahrer ausgesetzt ist, direkt unter Verwendung eines linearen Zusammenhangs zwischen Müdigkeit und Helligkeit zur Bestimmung der den Fahrerzustand repräsentierenden Größe verwendet wird.

## Claims

1. Method for determining a variable representing the tiredness of the driver of a vehicle, wherein the variable is determined independently of the time of day, at least on the basis of another variable, said other variable representing the brightness to which the driver is exposed,
**characterized in that**
the variable representing the brightness to which the driver is exposed is used directly to determine the variable representing the driver's state using a linear relationship between tiredness and brightness.

2. Method according to Claim 1,
**characterized in that**
the variable representing the brightness to which the driver is exposed is determined using a temporal filter, in particular a lag element.

3. Method according to Claim 1,
**characterized in that**
the brightness is measured in the form of the illuminance.

4. Method according to Claim 1,
**characterized in that**
light sensors are used for determining the brightness.

5. Method according to Claim 1,
**characterized in that**
the transverse guiding behaviour, in particular the steering behaviour of the driver, is included in the determination of the variable representing the driver's state.

6. Method according to Claim 1,
**characterized in that**
the longitudinal guiding behaviour, in particular the pedal actuation by the driver, is included in the determination of the variable representing the driver's state.

7. Method according to Claim 1,
**characterized in that**
the ability of the driver to keep the vehicle within one lane as far as possible is included in the determination of the variable representing the driver's state.

8. Method according to Claim 1,
**characterized in that**
the operation of operating elements, for example of the indicator and/or the window lift, by the driver is included in the determination of the variable representing the driver's state.

9. Method according to Claim 1,
**characterized in that**
the interaction of the driver with the vehicle via HMI and infotainment systems, such as an integrated navigation system and/or a voice control and/or speech output, is included in the determination of the variable representing the driver's state.

10. Method according to Claim 1,
**characterized in that**
a further variable that is established by way of a vehicle interior camera for observing the driver is included in the determination of the variable representing the driver's state.

11. Method according to Claim 1,
**characterized in that**
another variable that represents at least one physiological state of the driver is included in the determination of the variable representing the driver's state.

12. Method according to Claim 11,
**characterized in that**
the physiological variables of the driver are the body temperature and/or the heart rate.

13. Method according to Claim 11,
**characterized in that**
sensors are used to determine the physiological variables, wherein these sensors are attached in the rear-view mirror and/or in the driver's seat and/or in the driver's seat belt and/or in the steering wheel.

14. Apparatus for determining a variable representing the tiredness of the driver of a vehicle, wherein the apparatus is configured in such a way that the variable is determined independently of the time of day, at least on the basis of another variable, said other variable representing the brightness to which the driver is exposed,
**characterized in that**
the apparatus is configured in such a way that the variable representing the brightness to which the driver is exposed is used directly to determine the variable representing the driver's state using a linear relationship between tiredness and brightness.

## Revendications

1. Procédé de détermination d'une grandeur représentant la fatigue du conducteur d'un véhicule, la grandeur étant déterminée indépendamment de l'heure au moins à l'aide d'une autre grandeur qui représente la luminosité à laquelle est exposé le conducteur,
**caractérisé en ce que**
la grandeur qui représente la luminosité à laquelle est exposé le conducteur est utilisée directement pour déterminer la grandeur représentant l'état du conducteur en employant une relation linéaire entre la fatigue et la luminosité.

2. Procédé selon la revendication 1, **caractérisé en ce que** la grandeur qui représente la luminosité à laquelle est exposé le conducteur est déterminée en utilisant un filtre temporel, notamment un élément à temps de retard.

3. Procédé selon la revendication 1, **caractérisé en ce que** la luminosité est mesurée sous la forme de l'intensité d'éclairage.

4. Procédé selon la revendication 1, **caractérisé en ce que** des capteurs de lumière sont utilisés pour déterminer la luminosité.

5. Procédé selon la revendication 1, **caractérisé en ce que** le comportement de guidage transversal, notamment le comportement de direction du conducteur, intervient dans la détermination de la grandeur représentant l'état du conducteur.

6. Procédé selon la revendication 1, **caractérisé en ce que** le comportement de guidage longitudinal, notamment l'actionnement de la pédale par le conducteur, intervient dans la détermination de la grandeur représentant l'état du conducteur.

7. Procédé selon la revendication 1, **caractérisé en ce que** la capacité du conducteur à maintenir le véhicule autant que possible à l'intérieur d'une voie de circulation intervient dans la détermination de la grandeur représentant l'état du conducteur.

8. Procédé selon la revendication 1, **caractérisé en ce que** l'actionnement d'éléments de commande par le conducteur, par exemple du clignotant et/ou du lève-vitre, intervient dans la détermination de la grandeur représentant l'état du conducteur.

9. Procédé selon la revendication 1, **caractérisé en ce que** l'interaction du conducteur avec le véhicule par le biais de systèmes HMI et d'infodivertissement, comme un système de navigation intégré et/ou une commande vocale et/ou une sortie vocale, intervient dans la détermination de la grandeur représentant l'état du conducteur.

10. Procédé selon la revendication 1, **caractérisé en ce qu'**une grandeur supplémentaire intervient dans la détermination de la grandeur représentant l'état du conducteur, laquelle est déterminée par le biais d'une caméra d'habitacle de véhicule destinée à l'observation du conducteur.

11. Procédé selon la revendication 1, **caractérisé en ce qu'**une autre grandeur, laquelle représente au moins un état physiologique du conducteur, intervient dans la détermination de la grandeur représentant l'état du conducteur.

12. Procédé selon la revendication 11, **caractérisé en ce que** les grandeurs physiologiques du conducteur sont la température corporelle et/ou la fréquence cardiaque.

13. Procédé selon la revendication 11, **caractérisé en ce que** des capteurs sont utilisés pour déterminer les grandeurs physiologiques, ces capteurs étant montés dans le rétroviseur et/ou dans le siège de conducteur et/ou dans la ceinture de sécurité du conducteur et/ou dans le volant de direction.

14. Dispositif de détermination d'une grandeur représentant la fatigue du conducteur d'un véhicule, le dispositif étant configuré de telle sorte que la grandeur est déterminée indépendamment de l'heure au moins à l'aide d'une autre grandeur qui représente la luminosité à laquelle est exposé le conducteur,
**caractérisé en ce que**
le dispositif est configuré de telle sorte que la grandeur qui représente la luminosité à laquelle est exposé le conducteur est utilisée directement pour déterminer la grandeur représentant l'état du conducteur en employant une relation linéaire entre la fatigue et la luminosité.
